Europäisches Patentamt

(19) **European Patent Office**

**Office européen des brevets**

(11) Publication number: **0 148 709**
A1

## (12) EUROPEAN PATENT APPLICATION

(21) Application number: **84420082.4**

(22) Date of filing: **04.05.84**

(51) Int. Cl.⁴: **A 61 K 33/42,** A 01 N 59/26
// (A61K33/42, 33:00, 31:19)

(30) Priority: **09.01.84 US 569191**

(43) Date of publication of application: **17.07.85**
**Bulletin 85/29**

(84) Designated Contracting States: **AT BE CH DE FR GB IT**
**LI LU NL SE**

(71) Applicant: **CAPETROL INTERNATIONAL, INC.,**
**3500 West Robinson St Suite 204, Norman**
**Oklahoma 73070 (US)**

(72) Inventor: **Garcia, Silverio M., 2416 Osborne Drive,**
**Norman Oklahoma 73069 (US)**

(74) Representative: **Laurent, Michel, 20 rue Louis Chirpaz**
**Boîte postale no. 32, F-69131 Ecully Cedex (FR)**

(54) **Improved germicide composition.**

(57) Pharmaceutical agents useful as germicides in the treatment of infected tissue, the pharmaceutical agent comprising from about 1 to 100 weight percent of an aqueous acidic composition having a pH value of less than 1 and from 0 to about 99 weight percent of a carrier vehicle. The aqueous acidic compositions are prepared by mixing from about 45 to about 80 weight percent hydrochloric acid with from about 20 to about 55 weight percent phosphoric acid for a period of time effective to produce a substantially homogeneous acidic mixture; admixing the acidic mixture with an effective amount of water to produce an aqueous acidic mixture containing at least about 70 weight percent water; and admixing an effective minor amount of a hydroxy carboxylic acid into the aqueous acidic mixture.

IMPROVED GERMICIDE COMPOSITION

Background of the Invention

1. Field of the Invention

The present invention relates generally to pharmaceutical agents, and more particularly, but not by way of limitation, to improved germicides having pH values of less than about 1. In one aspect the present invention relates to a virucidal pharmaceutical agent comprising from about 1 to about 100 weight percent of an aqueous acidic composition having a pH value of less than about 1 and from 0 to about 99 weight percent of a carrier vehicle for the aqueous acidic composition. In another aspect the present invention relates to a method for topically treating infected tissue of human beings and animals using a pharmaceutical agent comprising from about 1 to about 100 weight percent of an aqueous acidic composition having a pH value of less than about 1 and from 0 to about 99 weight percent of a carrier vehicle for the aqueous composition.

2. Discussion of Prior Art

Throughout history man has healed himself and his animals against disease as best he could, often reaching out blindly toward the resource of nature, but sometimes using elaborate pharmaceutical theories, techniques and implements. In recent years much progress has been made in the use of pharmaceutical agents for treatment of man and animals. Most of the success has bee.. on the use of pharmaceutical agents in the treatment of bacterial infections, while only partial success has generally been achieved on infections caused by viruses, fungus or yeast. In addition, the pharmaceutical agents heretofore known tend to be specific to certain types or strains of bacteria, virus, fungi or yeast; and the treatment of such cultural strains requires extended periods of time and often substantial cost. Thus, new and improved pharmaceutical agents are constantly being sought which have a wide range of nonspecific topical application for treatment of infections caused by various cultural strains, such as bacteria, virus, fungus or yeast.

## Summary of the Invention

The present invention provides an improved pharmaceutical agent useful as a germicide in the topical treatment of infections caused by bacteria, virus, fungus or yeast. Broadly, the improved germicide comprises from about 1 to 100 weight percent of an aqueous acidic composition and having a pH value of less than about 1 and from 0 to about 99 weight percent of a carrier vehicle compatible with the aqueous acidic composition.

The aqueous acidic compositions employed as germicides in accordance with the present invention, either per se or in combination with a carrier vehicle, are prepared by (a) mixing from about 45 to about 80 weight percent hydrochloric acid with from about 20 to about 55 weight percent phosphoric acid for a period of time effective to produce a substantially homogeneous acidic mixture, (b) admixing the substantially homogeneous acidic mixture into an effective amount of water to produce an aqueous acidic mixture, and (c) admixing an effective minor amount of a hydroxy carboxylic acid into the aqueous acidic mixture to produce the aqueous acidic composition.

An object of the present invention is to provide an improved pharmaceutical agent having broad application in the treatment of infection, whether such infection is caused by bacteria, virus, fungus or yeast.

Another object of the present invention is to provide an improved method for the topical treatment of infected tissue.

Other objects, advantages, and features of the present invention will become apparent from the following detailed description when read in conjunction with the appended claims.

## Description of Preferred Embodiments

The present invention provides an improved pharmaceutical agent for the treatment of infected tissue, both of man and animals, whether the infection is caused by bacteria, virus, fungus or yeast. Broadly, the pharmaceutical agent comprises from about 1 to about 100 weight percent of an aqueous acidic composition having a pH value of less than about 1 and from 0 to about 99 weight percent of a carrier vehicle compatible with the aqueous acidic composition. In one aspect, the present invention provides a method for topically treating infected tissue employing the improved and unique pharmaceutical agents containing an effective amount of the aqueous acidic composition as a critical component.

The aqueous acidic compositions employed as the pharmaceutical agent or germicide in accordance with the present invention, either per se or in combination with a carrier vehicle, are prepared from selected inorganic and organic acids. The exact structure and function of the aqueous acidic composition is not known other than its apparent abilities to inactivate the source of the infection, such as a virus, when the aqueous acidic composition is applied to the area of infected tissue. However, it should be noted that while the aqueous acidic compositions appear to inactivate the source causing the infection in the tissue, the aqueous acidic compositions, per se and in combination with a carrier vehicle, appear to be substantially inert to healthy tissue and do not attack or damage the healthy tissue surrounding infected tissue.

As previously stated, the exact structure of the aqueous acidic compositions is not known. However, one theory is that during the preparation of the aqueous acidic compositions, a high concentration of hydrogen ions (H+) are released into and remain present in the aqueous acidic composition. This high concentration of hydrogen ions (H+) would account for the low pH values of the aqueous acidic compositions.

- 5 -

The term "pH value" is generally employed as a means for expressing the degree of acidity or basicity of a solution. For example, at normal temperature a solution, such as pure distilled water, has a pH of about 7; whereas a one-tenth normal solution of hydrochloric acid (approximately 3.65 grams hydrochloric acid per liter of water) has a pH near 1 and is considered strongly acidic. Thus, the aqueous acidic compositions having pH values of less than about 1 are considered strongly acidic in nature.

The aqueous acidic compositions are prepared by a process wherein the ingredients are believed critical. All mixing and storage containers employed in the production of the aqueous acidic compositions are desirably fabricated of a substance that is acid resistant, such as stainless steel, plastic, fiberglass, glass and the like. It is also desirable that all containers used in the preparation of the aqueous acidic compositions be provided with covers for safety reasons, especially the container in which the hydrochloric acid and phosphoric acid are mixed, and to keep foreign material out of the acidic composition. Further, because strong fumes are emitted upon mixing the hydrochloric acid and the phosphoric acid, care should be exercised in the mixing of the two components to ensure that the mixing step is carried out in a well ventilated area or hood.

The order of addition of the ingredients used in the formulation of the aqueous acidic composition can be varied without substantially altering the properties of the acidic composition. However, especially desirable results have been obtained when the aqueous acidic composition is prepared in accordance with the following procedure. The initial step in preparing the aqueous acidic composition comprises admixing from about 45 to about 80 weight percent hydrochloric acid with from about 20 to about 55 weight percent phosphoric acid for an effective period of time to produce a substantially homogeneous acidic mixture. The time required to mix the hydrochloric acid and the phosphoric acid so as to provide a substantially uniform acidic mixture can vary widely; and the mixing time or period will generally depend upon the rate of addition of the two components, the amount of the two components, the rate or speed of agitation and the like.

The homogeneous acidic mixture formed from the hydrochloric acid and the phosphoric acid is then admixed with an effective amount of water to provide an aqueous acidic mixture. The amount of water employed in the formation of the aqueous acidic mixture can vary widely but it is an amount sufficient to desirably provide at least about 70 weight percent water in the aqueous acidic composition. The aqueous acidic mixture is thoroughly stirred to ensure substantially complete dispersion of the homogeneous acidic mixture of the hydrochloric acid and the phosphoric acid into the water and to provide a substantially uniform aqueous acidic mixture.

While maintaining agitation on the aqueous acidic mixture, from about 2 to about 20 weight percent of a hydroxy carboxylic acid and from about 0 to about 20 weight percent of a dicarboxylic acid are admixed with the aqueous acidic mixture to produce the aqueous acidic composition. The hydroxy carboxylic acid appears to be a critical ingredient in the formation of the aqueous acidic compositions; whereas the dicarboxylic acid appears to be an optional ingredient. However, desirable results have been obtained when the dicarboxylic acid and the hydroxy carboxylic acid are both employed in the formulation of the aqueous acidic composition.

The amounts of hydroxy carboxylic acid and dicarboxylic acid incorporated into the aqueous acidic mixture of the hydrochloric acid and the phosphoric acid can vary widely within the range set forth hereinabove. However, the optimum amounts of the hydroxy carboxylic acid and the dicarboxylic acid admixed with the aqueous acidic mixture are the amounts required to provide from about 2 to about 10 weight percent of the hydroxy carboxylic acid and from about 1 to about 10 weight percent of the dicarboxylic acid in the aqueous acidic composition.

When incorporating the dicarboxylic acid into the aqueous acidic mixture in the formulation of the aqueous acidic composition the order of addition of the dicarboxylic and the hydroxy carboxylic acid is not critical. However, desirable results have been obtained when the dicarboxylic acid is introduced into the aqueous acidic mixture following the addition of the hydroxy carboxylic acid.

Any suitable hydroxy carboxylic acid can be employed in the preparation of the aqueous acidic composition. Typical of such hydroxy carboxylic acids are citric acid, tartaric acid, malic acid and the like. However, especially desirable results have been obtained when the hydroxy carboxylic acid added to the aqueous acidic mixture in the formulation of the aqueous acidic compositions is citric acid.

Any suitable dicarboxylic acid can also be employed in the preparation of the aqueous acidic composition. Typical of such dicarboxylic acids are oxalic acid, malonic acid, succinic acid, glutaric acid, adipic acid and the like. However, when employing a dicarboxylic acid in the formulation of the aqueous acidic composition, desirable results have been obtained when the dicarboxylic acid added to the aqueous acidic mixture is oxalic acid.

The aqueous acidic composition so produced will desirably contain at least about 70 weight percent water, preferably from about 70 to about 90 weight percent water. More desirably, the aqueous acidic compositions will contain from about 75 to about 80 weight percent water. Thus, depending upon the amounts of hydroxy carboxylic acid and dicarboxylic acid added to the aqueous acidic mixture of the hydrochloric acid and phosphoric acid, as well as the amount of water initially added to the acidic mixture to form the aqueous acidic mixture, it may be desirable to further dilute the aqueous acidic composition with an effective amount of water to insure that the concentration of water in the aqueous acidic composition is from about 70 to about 90 weight percent, and more desirably from about 75 to about 80 weight percent. In those instances where it is determined that the amount of water present in the aqueous acidic composition is less than the specified amount, the aqueous acidic composition is admixed with an effective amount of water so as to provide the aqueous acidic composition with the desired amount of water as specified above.

The aqueous acidic composition prepared by the process described above is a substantially colorless liquid having an appearance similar to water. Further, the aqueous acidic composition, while possessing strong acidic characteristics (i.e. a pH value of about 0.49), is substantially inert to healthy human tissue, clothing, metal, polymeric materials and the like.

When employing a carrier vehicle in the formulation of the pharmaceutical agent or germicide comprising the aqueous acidic composition any suitable carrier vehicle which is compatible with the aqueous acidic component can be employed provided such carrier vehicle is safe for human use. Typical examples of suitable carrier vehicles which may be admixed with the aqueous acidic composition to provide improved germicides are water, alcohols such as ethanol and propanol, and liquid detergent compositions.

The amount of a carrier vehicle employed in the formulation of the pharmaceutical agent or germicide containing the aqueous acidic composition can vary widely and will be dependent in a large degree to the specific application or use of the pharmaceutical agent or germicide. Generally, however, the pharmaceutical agents or germicides of the present invention containing a carrier vehicle will consist essentially of from about 1 to 75 weight percent of the aqueous acidic composition having a pH value of less than about 1 and from about 25 to 99 weight percent of a carrier vehicle.

The pharmaceutical agent or germicide described above can be employed as a topical agent in the treating of infected tissue of both man and animals. As previously stated, the germicide can comprise the aqueous acidic composition having a pH value of less than about 1, per se, or a mixture containing from about 1 to 100 weight percent (desirably from about 1 to about 75 weight percent) of the aqueous acidic composition and from 0 to 99 weight percent (desirably from about 25 to about 99 weight percent) of a suitable carrier vehicle for the aqueous acidic composition.

When employing the pharmaceutical agent or germicides of the present invention as topical agents in the treating of infected tissue an effective amount of germicide is applied to the infected tissue to substantially coat the infected tissue and a portion of healthy tissue surrounding the infected tissue. Any suitable method can be employed in the application of the germicide to the infected tissue, such as a cotton swab, a spray, and the like. The length of time between applications of the germicide to the infected tissue can vary widely and will be dependent, to a large extent, on the degree of infection in the tissue, and the composition of the germicide. For example, when applying the aqueous acidic composition, per se, or a mixture containing the aqueous acidic composition and the carrier vehicle, as a pharmaceutical agent or germicide to infected tissue, the application of the germicide should be repeated at about 24 hour intervals until the infected tissue is rendered substantially dormant.

The exact function of the germicide containing the aqueous acidic composition having a pH value of less than about 1 (i.e. 0.49) in the treating of infected tissue is not known, especially in view of its apparent nonspecificity to particular infection causing agents. Normally a solution with this pH would immediately be considered not safe for human use due to the extreme acidity of the acidic composition, potential precipitation of protein and corrosive action upon skin. However, preliminary laboratory testing indicated that this substance, even with its low pH, does not precipitate protein while reacting actively with bicarbonate in the release of carbon dioxide in a serum specimen. This truly indicates unique properties of the aqueous acidic composition.

In further testing to evaluate its potential virucidal activities, that is for potential use in herpes, the virucidal activity of the aqueous acidic composition were compared to normal detergent activity. The minimum criteria for classification as a detergent having virucidal activities requires a "factor of activity of $10^3$. The formulation containing the aqueous acidic composition surpassed this minimum requirement and gave an activity factor of $10^{4.5}$. This indicated a magnitude of activity of 1.5 times the minimum required amount as a logarithmic function.

Furthermore, studies were performed to evaluate "drug-like" activities of the aqueous acidic composition. Cells were allowed to equilibrate with the herpes virus and time was allowed for the herpes virus to enter appropriate cells. Treatment with serial doses of a pharmaceutical agent comprising the aqueous acidic composition and a carrier vehicle (water) did show activities that arrested the effect of the virus on the cells, showing at least the ability to arrest further activity of the herpes virus in the particular cell culture. The initial findings indicate that a pharmaceutical agent containing the aqueous acidic composition having a pH value of less than about 1 is virucidal for the herpes virus and does possess some qualities that make it unique as a potential chemical for use in the treatment for symptomatic relief of the herpes virus.

In order to more fully describe the present invention, the following examples are set forth. However, it is to be understood that the examples are for illustrative purposes and are not to be construed as limiting the scope of the present invention as defined in the appended claims.

0148709

## EXAMPLE I

### Preparation of Aqueous Acidic Composition

3.57 pounds of hydrochloric acid and 2.08 pounds of phosphoric acid were added to a container and the acids were stirred to produce a substantially homogeneous acidic mixture. During the mixing of the hydrochloric acid and the phosphoric acid fumes were generated. ·Thus, the mixing of the hydrochloric acid and the phosphoric acid was carried out in a ventilated area.

16.6 pounds of clean water was then placed into a second container. 5.64 pounds of the hydrochloric-phosphoric mixture was added to the water in the second container. The resulting aqueous acidic solution was mixed thoroughly. Thereafter, 1.08 pounds of powdered citric acid and 0.75 pounds of powdered oxalic acid were admixed into the aqueous acidic mixture to produce an aqueous acidic composition.

The aqeuous acidic composition was then diluted by admixing 24.07 pounds of the aqueous acidic composition with 16.6 pounds of clean water in a third container. The aqueous acidic composition and water were thoroughly stirred and provided 4.85 gallons (40.7 pounds) of the aqueous acidic composition.

It should be noted that the mixing and storage containers employed were plastic containers which were substantially acid resistant. Further, all containers were covered for safety reasons and to prevent foreign materials from being injected into the aqueous acidic composition. The aqueous acidic composition so prepared had a pH value of 0.49 and was substantially inert to healthy human skin and clothing.

## EXAMPLE II

BHK-21 Cells (a fibroblastic cell line) were seeded into 96 well microtiter plates at a concentration of 1.6 x $10^5$ cells/ml and cultured overnight. The virus solution, Type I Herpes Simplex Virus (strain C-148), was removed from a -70°C freezer and thawed. The virus suspension was mixed with either an equal volume of cell culture medium or an equal volume of the aqueous acidic composition prepared in accordance with Example I and incubated for 10 minutes at room temperature. After the incubation period a series of log dilutions of each solution was made. The cytotoxicity control was performed in a similar manner. Eight one-log dilutions were made of each solution. After the dilutions were prepared, medium was decanted from the cells in the microplate and test solutions added in 0.2 ml volumes to four (4) cups for each dilution. The microplates were then sealed and incubated for approximately 72 hours at 37°C. After incubation, each cup was microscopically observed for development of virus-induced cytopathic effect (CPE) or cell change resulting from the test solution. The results of these experiments are set forth in Table IIA.

## TABLE IIA

EFFECT OF INCUBATING A 50% SOLUTION OF THE AQUEOUS ACIDIC COMPOSITION OF EXAMPLE I FOR 10 MINUTES AT ROOM TEMPERATURE ON THE SURVIVAL OF BHK CELLS AND TYPE I HERPES SIMPLEX VIRUS (STRAIN C-148).

| Dilution of Solution | Virus + Product of Example I | Virus + (Virus Control) | Cytotoxicity Control | Remarks |
|---|---|---|---|---|
| $10^{-1}$ | Not determined | Not determined | - - - - - - | Cells appeared to be alive, but looked abnormal. Medium acidic. |
| $10^{-2}$ | - - - - - -(1) | + + + + + + | - - - - - - | Extensive precipitation of medium materials. Medium acidic. |
| $10^{-3}$ | 0 0 0 0 0 0 | + + + + + + | 0 0 0 0 0 0 | Highest concentration of the aqueous acidic composition prepared in accordance with Example I that was not appreciably acidic. |
| $10^{-4}$ | 0 0 0 0 0 0 | + + + + + + + | 0 0 0 0 0 0 | |
| $10^{-5}$ | 0 0 0 0 0 0 | + + + + + + | 0 0 0 0 0 0 | |
| $10^{-6}$ | 0 0 0 0 0 0 | + + + + + + | 0 0 0 0 0 0 | |
| $10^{-7}$ | 0 0 0 0 0 0 | 0 0 0 0 0 0 | 0 0 0 0 0 0 | |
| $10^{-8}$ | 0 0 0 0 0 0 | 0 0 0 0 0 0 | 0 0 0 0 0 0 | |
| Titer(2) | No detectable virus | $10^{6.5}/0.2ml$ $TCLD_{50}$ | $10^{2.5}/0.2ml$ $TCLD_{50}$ | |

Virus Inactivation(3) = $10^{6.5} - 10^{2.5} = 10^{4.0}$

(1) Impossible to observe cell morphology as a result of a precipitant of medium constituents.
(2) $TCID_{50}$ = Tissue Culture 50% infectious dose. $TCLD_{50}$ = Tissue culture 50% lethal dose.
(3) A virus inactivation score of $10^3$ is considered the minimum active level for a disinfectant by the EPA.

In order to determine the effect of the aqueous acidic composition as prepared in accordance with Example I and using the procedure of Example IIA on the viability of BHK-21 Cells in tissue culture a spectrophotometric assay of cell viability was conducted using natural red as the stain. Natural red is a stain which normal healthy cells absorb. Cells exposed to the 1:20 and 1:200 dilutions were minimally active, although it is possible that the cells might survive if their medium were replaced with fresh medium. The results of these experiments are set forth in Table IIB.

## TABLE IIB

| Dilution of Aqueous Acidic Composition | Microscopic Observation of Cells | Neutral Red Dye Uptake (% of Control) |
|---|---|---|
| 1:20 | Appeared alive but exhibited abnormal morphology | 9.7 |
| 1:200 | Impossible to observe cell morphology as a result of precipitation of medium constituents | 9.7 |
| 1:2000 | Cells appeared normal | 94.0 |

The data set forth in Table IIA and IIB clearly indicate the virucidal properties of the aqueous acidic composition prepared in accordance with Example I for the Type I Herpes Virus.

## EXAMPLE III

The aqueous acidic composition prepared in accordance with Example I was applied to the leg of an individual having an infected area diagnosed to be a ringworm. Four applications of the germicide were applied to the infection over a time interval of about 18 hours. After the first application a substantial improvement in the coloration of the tissue surrounding the ringworm and the ringworm itself was detected. However, because of the excess treatment, a slight burn to the tissue in the treated area was noticed at the end of 18 hours. Treatment was stopped for 48 hours and the germicide was again administered to the infected area. Within 48 hours of the last treatment the slight burn caused to the skin had dissipated and the ringworm cured.

## EXAMPLE IV

The aqueous acidic composition prepared in accordance with Example I was applied to excema on a person's foot. Application to the excema was at the rate of four times in an 18 hour period. The excess application resulted in a slight burn to the excema-weakened skin. Thus, treatment was stopped for 24 hours and then treatment proceeded on a once a day basis by applying an effective amount of the germicide to the excema infected area and skin surrounding such area. No further spreading of the excema was detected and after about two weeks of treatment a substantial improvement in the excema was detected.

## EXAMPLE V

Cankor sores in the mouth were treated using a mouth wash containing 100% of the aqueous acid composition prepared in Example I. Due to the damaged membrane surface at the site of the cankor sores in the mouth, the germicide gave the person a burning sensation for a short period of time, substantially similar to the burning sensation experienced with an alcohol based mouthwash. After one treatment, and within 24 hours after such treatment, the cankor sores disappeared and there was no visible damage to healthy oral membranes.

## EXAMPLE VI

A sore throat, a result of an infection, was treated with a germicide comprising about 15 weight percent of the aqueous acidic composition prepared in Example I and 85 weight percent water. The inflamed and swollen area around the tonsils was painted with the germicide with a cotton swab. 24 hours after the treatment the area was inspected and the inflation, swelling and soreness had dissipated.

## EXAMPLE VII

About 15 weight percent of the aqueous acidic composition prepared in accordance with Example I was admixed with about 85 weight percent of a liquid detergent to provide a germicide. The germicide was applied to the hands as a surgical scrub. Upon scrubbing of the hands sudsing of the composition was achieved and the hands were substantially clean and free of foreign materials.

The improved pharmaceutical agent or germicide set forth hereinbefore, whether comprising the aqueous acidic composition, per se, or a mixture of the aqueous acidic composition and up to 99 weight percent of the carrier vehicle provide a new array of pharmaceutical agents having special properties which can be employed to topically treat infected tissue without fear of damage to healthy tissue. Because of the unique properties of the aqueous acidic compositions the germicides of the present invention containing the aqueous acidic compositions appear to have wide application to cauterize and destroy infection causing agents by rendering such agents incapable of propogating, whether such infection causing agents are classified as a bacteria, virus, fungi or yeast.

It is clear that the present invention is well adapted to carry out the objects and attain the ends and advantages mentioned herein as well as those inherent in the invention. While presently preferred embodiments of the invention have been described for purposes of this disclosure, numerous changes may be made which will readily suggest themselves to those skilled in the art and which are accomplished within the spirit disclosed and as defined in the appended claims.

CLAIMS

1.    An improved germicide comprising :

(a)   from about 1 to 100 weight percent of an aqueous acidic composition having a pH value of less than about 1, the aqueous acidic composition prepared by the steps of :

(1)   mixing from about 45 to about 80 weight percent hydrochloric acid with from about 20 to about 55 weight percent phosphoric acid for a period of time effective to produce a substantially homogeneous acidic mixture ;

(2)   admixing the acidic mixture with an effective amount of water to produce an aqueous acidic mixture containing at least about 70 weight percent water ; and

(3)   admixing an effective minor amount of a hydroxy carboxylic acid into the aqueous acidic mixture to produce the aqueous acidic composition ;

and,

(b)   from 0 to about 99 weight percent of a carrier vehicle compatible with the aqueous acidic composition.


2.    The improved germicide of claim 1 wherein the preparation of the aqueous acidic composition further comprises admixing from 2 to about 20 weight percent of a carboxylic acid into the aqueous acidic mixture.

3.    The improved germicide of claim 2 wherein the carrier vehicle is selected from the group consisting of water, ethanol, propanol and liquid detergent compositions.

4.    The improved germicide of claim 2 wherein the hydroxy carboxylic acid employed in the formulation of the aqueous acidic composition is selected from the group consisting in citric acid and oxalic acid.

5.    The improved germicide of claim 1 wherein the preparation of the aqueous acidic composition as set forth in step (a) further comprises :

(4)    admixing up to about 20 weight percent of a dicarboxylic acid into the aqueous acidic mixture ; and

(5)    diluting the aqueous acidic composition with an effective amount of water to provide from about 70 to about 90 weight percent water in the aqueous acidic composition.

6.    The improved germicide of claim 5 wherein the carboxylic acid is incorporated into the acidic mixture in a amount sufficient to provide from about 1 to about 10 weight percent of the carboxylic acid in the aqueous acidic composition.

7.    The improved germicide of claim 6 wherein the dicarboxylic acid employed in the formulation of the aqueous acidic composition is oxalic acid.

8.    The improved germicide of claim 7 wherein the germicide comprises from about 1 to about 75 weight percent of the aqueous acidic composition and from about 25 to about 99 weight percent of the carrier vehicle.

9.    The improved germicide of claim 8 wherein the diluent is selected from the group consisting of water, ethanol, isopropanol and liquid detergent compositions.

CLAIMS

1. A method for preparing a germicide composition, characterized by the fact that the germicide is prepared by the steps of :

(a) mixing from about 45 to about 80 weight percent hydrochloric acid with from about 20 to about 55 weight percent phosphoric acid for a period of time effective to produce a substantially homogeneous acidic mixture ;

(b) admixing the acidic mixture with an effective amount of water to produce an aqueous acidic mixture containing at least about 70 weight percent water ; and

(c) admixing an effective minor amount of a hydroxy carboxylic acid into the aqueous acidic mixture to produce the aqueous acidic composition.

2. The method of claim 1 wherein the germicide further comprises from about 1 to about 99 weight percent of a carrier vehicle compatible with the aqueous acidic composition.

3. The method of claim 2 wherein the carrier vehicle is selected from the group consisting of water, ethanol, isopropanol and liquid detergent compositions.

4. The method of claim 1, wherein the preparation further comprises admixing up to about 20 weight percent of a dicarboxylic acid into the aqueous acidic mixture.

5. The method of claim 4 wherein the aqueous acidic composition contains from about 70 to about 90 weight percent water.

6. The method of claim 4 wherein the aqueous acidic composition is diluted with an effective amount of water to provide from about 70 to about 90 weight percent water in the aqueous acidic composition.

7. The method of claim 4 wherein the hydroxy carboxylic acid employed in the formulation of the aqueous acidic composition is citric acid, and the citric acid is incorporated into the acidic mixture in an amount sufficient to provide from about 2 to about 10 weight percent citric acid in the aqueous acidic composition.

8. The method of claim 7 wherein the dicarboxylic acid admixed with the aqueous acidic mixture is an amount sufficient to provide from about 1 to about 10 weight percent of the dicarboxylic acid in the aqueous acidic composition.

9. The method of claim 8 wherein the dicarboxylic acid is oxalic acid.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 85, no. 4, 26th July 1976, page 94, no. 23059v, Columbus, Ohio, US; & JP - A - 76 12390 (K. TAKADA et al.) 30-01-1976 * Abstract * | 1-9 | A 61 K 33/42 A 01 N 59/26 // (A 61 K 33/42 A 61 K 33:00 A 61 K 31:19 ) |
| A | FR-A-1 169 512 (MONSANTO CHEMICALS LTD.) * Pages 2,3, abstract * | 1-9 | |
| A | DE-A-2 349 058 (HENKEL & CIE GmbH) * Page 14; claims 1-3 * | 1-9 | |
| A | FR-A- 901 397 (Dr. MADAUS & CO.) * Page 1, abstract no. 3 * | 1-9 | |
| A | FR-A-1 003 546 (IG FARBENINDUSTRIE AG) | 1-9 | |

TECHNICAL FIELDS SEARCHED (Int. Cl.4)

A 61 K
A 01 N

The present search report has been drawn up for all claims

| Place of search | Date of completion of the search | Examiner |
|---|---|---|
| THE HAGUE | 01-04-1985 | BRINKMANN C. |